(19) Europäisches Patentamt
European Patent Office
Office européen des brevets

(11) Publication number: **0 402 469 B1**

(12) **EUROPEAN PATENT SPECIFICATION**

(45) Date of publication of patent specification: **01.06.94**

(51) Int. Cl.5: **C07C 49/245**, A61K 31/12, C12N 9/99

(21) Application number: **89903276.7**

(22) Date of filing: **02.03.89**

(86) International application number: **PCT/JP89/00217**

(87) International publication number: **WO 89/08093 (08.09.89 89/21)**

(54) **CATECHOL COMPOUNDS, PROCESS FOR THEIR PREPARATION AND PHARMACEUTICAL PREPARATION CONTAINING SAME.**

(30) Priority: **02.03.88 JP 48955/88**
**05.07.88 JP 167064/88**

(43) Date of publication of application:
**19.12.90 Bulletin 90/51**

(45) Publication of the grant of the patent:
**01.06.94 Bulletin 94/22**

(84) Designated Contracting States:
**BE CH DE FR GB IT LI NL SE**

(56) References cited:
**JP-A- 6 092 230**
**JP-A- 6 112 642**
**JP-A-59 225 136**
**JP-A-60 178 837**
**JP-A-63 115 834**

**PATENT ABSTRACTS OF JAPAN, vol. 13, no. 262 (C-608)[3610], 16th June 1989;& JP-A-1 66 138**

(73) Proprietor: **TERUMO KABUSHIKI KAISHA**
**No. 44-1, Hatagaya 2-chome,**
**Shibuya-ku**
**Tokyo 151(JP)**

(72) Inventor: **OHNISHI, Hiroyuki Terumo Kabushiki Kaisha**
**44-1, Hatagaya 2-chome**
**Shibuya-ku**
**Tokyo 151(JP)**
Inventor: **SHIKATA, Yoshiyuki Terumo Kabushiki Kaisha**
**44-1, Hatagaya 2-chome**
**Shibuya-ku**
**Tokyo 151(JP)**
Inventor: **ENDO, Isamu Terumo Kabushiki Kaisha**
**44-1, Hatagaya 2-chome**
**Shibuya-ku**
**Tokyo 151(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 402 469 B1**

**PATENT ABSTRACTS OF JAPAN, vol. 14, no. 146 (C-704)[4089], 20th March 1990;& JP-A-2 17 146**

**PATENT ABSTRACTS OF JAPAN, vol. 10, no. 328 (C-383)[2384], 7th November 1986;& JP-A-61 137 834**

**PATENT ABSTRACTS OF JAPAN, vol. 12, no. 301 (C-521)[3148], 16th August 1988;& JP-A-63 72 625**

**PATENT ABSTRACTS OF JAPAN, vol. 12, no. 391 (C-537)[3238], 18th October 1988;& JP-A-63 139 124**

**Nihon Kagakukai-hen [Shin Jikken Kagaku Koza (vol. 14) Yuki Kagobutsu no Gosei to Hanno-II], 20 December 1977, Maruzen, pp. 851-853**

Inventor: **USHIJIMA, Hideto Terumo Kabushiki Kaisha**
**44-1, Hatagaya 2-chome**
**Shibuya-ku Tokyo 151(JP)**
Inventor: **ISOZAKI, Masashi Terumo Kabushiki Kaisha**
**44-1, Hatagaya 2-chome**
**Shibuya-ku Tokyo 151(JP)**

(74) Representative: **Gillard, Marie-Louise et al**
**Cabinet Beau de Loménie**
**158, rue de l'Université**
**F-75340 Paris Cédex 07 (FR)**

## Description

This invention relates to a novel catechol compound, a method for the production thereof, and medical preparations containing the compound. More particularly, this invention relates to a catechol compound, a method for the production thereof, and a 5-lipoxygenase action inhibitor and an ulcer depressant containing the compound.

Catechol compounds have already been disclosed. Reference could be made to :

1) US Patent 4 618 627, which relates to catechol derivatives and pharmaceutical compositions thereof for inhibiting anaphylaxis (SRS-A), which have the formula :

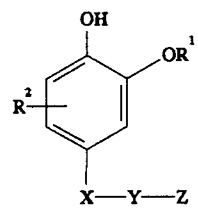

in which notably : $R_1$ is hydrogen, $R_2$ is hydrogen, X represents a straight chain or branched alkylene group having 1 to 15 C or a vinylene group, Y is a carbonyl, Z is a straight or branched alkyl group having 1 to 15 carbon atoms, the sum of the carbon atoms of X and Y being at least 3.

2) JP-A-61-12642 relates to compounds of formula :

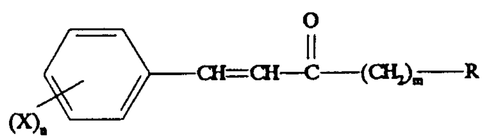

in which, among others, R is hydrogen, X is OH, n is 2, m is 1 to 7.

## Background Art

Leucotrienes such as leucotriene $C_4$ ($LTC_4$) and leucotriene $D_4$ ($LTD_4$) which are critical factors for allergy are biosynthesized from arachidonic acid by the action of 5-lipoxygenase.

It has been recently demonstrated that leucotrienes have their part in the crisis of not only allergy but also such morbid conditions as nephrities, and gastric ulcer.

It has been posed as a task, therefore, to seek out a substance effective in curbing the biosynthesis of leucotrienes and curing such diseases as caused by the leucotrienes. It has been also posed as a task to seek out a substance possessing an activity to depress various ulcers represented by gastric ulcer.

The inventors have synthesized various catechol compounds and pursued a diligent study on their activities to inhibit the action of 5-lipoxygenase and to resist the growth of ulcers. They have consequently found that the catechol compounds according with the present invention possess powerful activities to inhibit the action of 5-lipoxygenase and to resist the growth of ulcers. This invention has been perfected as the result. The catechol compounds of the present invention which possess the activities to inhibit the action of 5-lipoxygenase are useful for depressing the biosynthesis of leucotrienes and for curing nephritis, hepatitis, rheumatism, and gastric ulcer as well as allergic diseases such as asthma and rhinitis.

Further, the catechol compounds of this invention which possess the activity to resist the growth of ulcers are useful for the cure of various ulcers represented by gastic ulcer.

An object of this invention, therefore, is to provide novel catechol compounds, a method for the production thereof, and the agents containing the catechol compounds and used for inhibiting the action of 5-lipoxygenase and resisting the growth of ulcers.

Dislosure of the Invention

The object described above is accomplished by a catechol compound represented by the formula I:

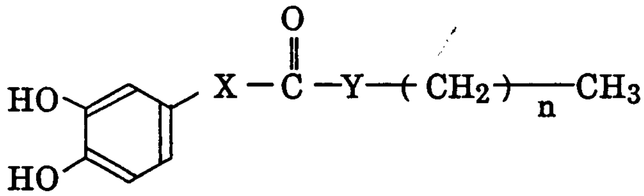

(I)

wherein X is one group selected from the class consisting of $-CH_2-CH_2-$ and -CH=CH-, Y is one group selected from the class consisting of -CH=CH- and $-CH_2-CZ-$ (where Z is =O, $<^{OH}_{H}$, or $<^{H}_{H}$), and n is an integer in the range of 2 to 8.

The object is also accomplished by a method for the production of a catechol compound represented by the formula Ia:

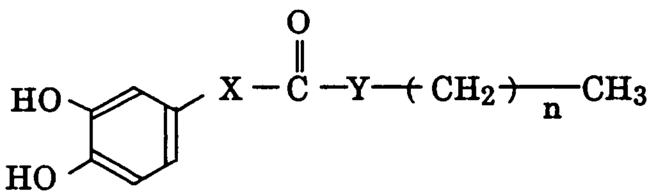

(Ia)

wherein X is -CH=CH-, Y is

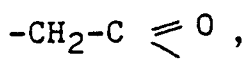

and n is an integer in the range of 2 to 8, which method comprises causing a benzylidene acetone compound represented by the formula II:

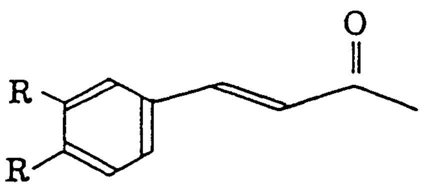

(II)

wherein R is methoxymethyloxy group, to react with at least one basic compound selected from the group consisting of alkali metal dialkyl amides, alkali metal dicycloalkyl amides, alkali metal alkoxides, alkyl alkalimetals, and alkali metal hexamethyl silazide thereby forming an elonate, subjecting the enolate to a reaction with an n-alkanoic acid imidazole amide having an alkyl group of 3 to 9 carbon atoms thereby forming an unsaturated β-diketone, and treating the unsaturated β-diketone with a mineral acid.

The object is further accomplished by a method for the production of a catechol compound represented by the formula Ib:

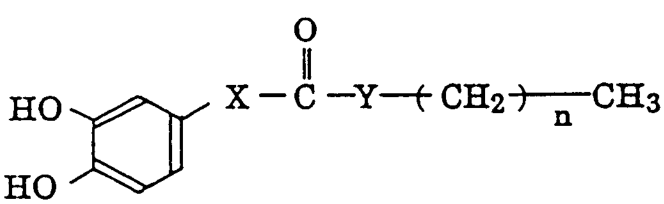

(Ib)

wherein X is $-CH_2-CH_2-$, Y is

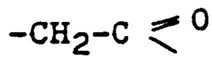 ,

and n is an integer in the range of 2 to 8, which method comprises causing a catechol compound represented by the formula Ia mentioned above to react with hydrogen in the presence of a reducing catalyst.

The object is also accomplished by a method for the production of a catechol compound represented by the formula Ic:

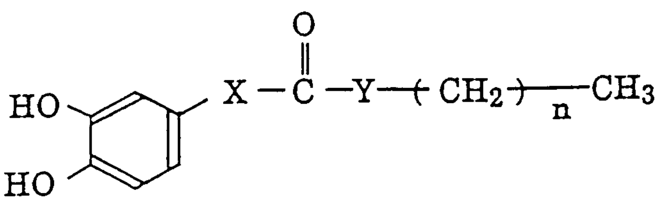

(Ic)

wherein X is $-CH=CH-$, Y is $-CH_2-CH(OH)-$, and n is an integer in the range of 2 to 8, which method comprises causing a benzylidene acetone compound represented by the formula II:

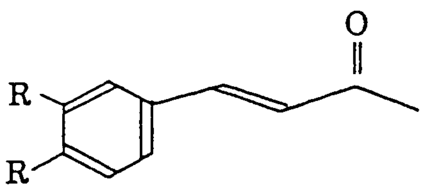

(II)

wherein R is methoxymethyloxy group, to react with at least one basic compound selected from the group consisting of alkali metal dialkyl amides, alkali metal dicycloalkyl amides, alkali metal alkoxides, alkyl alkali metals, and alkali metal hexamethyl silazide thereby forming an enolate, subjecting the enolate to a reaction with an n-alkane aldehyde thereby forming an unsaturated β-hydroxy ketone, and treating the unsaturated β-hydroxy ketone with titanium tetrachloride.

The object is further accomplished by a method for the production of a catechol compound represented by the formula Id:

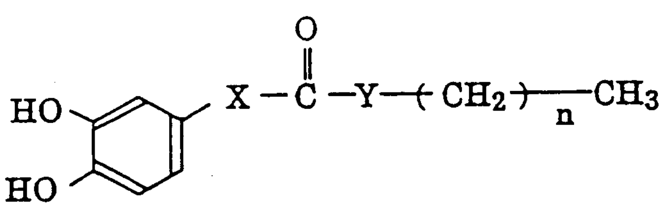

(Id)

wherein X is $-CH_2-CH_2-$, Y is $-CH_2-CH(OH)-$ and n is an integer in the range of 2 to 8, which method comprises causing a catechol compound represented by the formula Ic to react with hydrogen in the presence of a reducing catalyst.

The object are further accomplished by a method for the production of a catechol compound represented by the formula Ie:

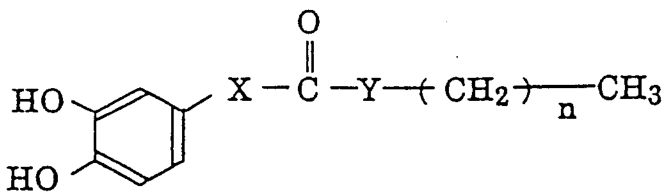

(Ie)

wherein X is $-CH_2-CH_2-$, Y for -CH = CH-, and n is an integer in the range of 2 to 8, which method comprises dehydrating a catechol compound represented by the formula Id in the presence of an acid.

The object is also accomplished by a 5-lipoxygenase action inhibiting agent having as an active component thereof a catechol compound represented by the formula I.

Best Mode for Carrying Out the invention

The catechol compounds of this invention are represented by the formula I:

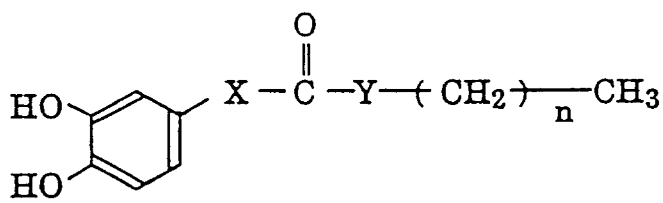

(I)

wherein X is one group selected from the class consisting of $-CH_2-CH_2-$ and -CH = CH-, Y is one group selected from the class consisting of -CH = CH- and $-CH_2-CZ-$ where Z is O, $<^{OH}_{H}$, or $<^{H}_{H}$, and n is an integer in the range of 2 to 8.

In the formula I, when n is an integer in the range of 2 to 8, Y is preferable to be

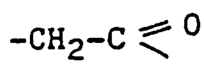

and, when n is 8, Y is preferable to be one group selected from the class consisting of $-CH_2-CH(OH)-$, -CH = HC-, and $-CH_2-CH_2-$.

In the catechol compounds mentioned above, a catechol compound represented by the formula Ia:

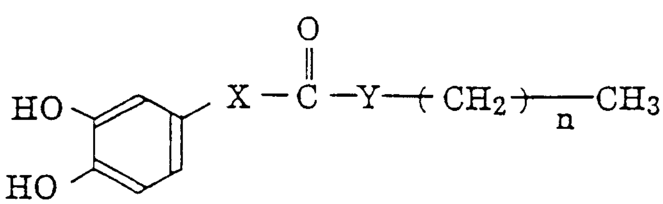

(Ia)

wherein X is -CH = CH-, Y is

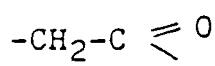 ,

and n is an integer in the range of 2 to 8, is obtained by causing a benzylidene acetone compound represented by the formula II:

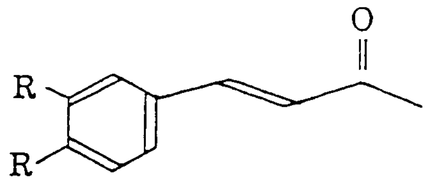

(II)

wherein R is methoxymethyloxy group, to react with at least one basic compound selected from the group consisting of alkali metal dialkyl amides, alkali metal dicycloalkyl amides, alkali metal alkoxides, alkyl alkali metals, and alkali metal hexamethyl silazide, preferably an alkali metal dialkyl amide, thereby forming an enolate, then causing the enolate to react with an n-alkane imidazole amide having an alkyl group of 3 to 9 carbon atoms thereby forming an unsaturated β-diketone, and treating the unsaturated β-diketone with a mineral acid.

The alkali metals which are usable for the formation of the basic compound include lithium, sodium, and potassium. Among other alkali metals mentioned above, lithium proves to be particularly preferable. The alkyl groups which are usable for the alkyl amides are those having 1 to 4 carbon atoms. The amount of the basic compound to be used is in the range of 1 to 2 mol equivalent weight, preferably 1.1 to 1.5 mol equivalent weight, based on 1 mol of a benzylidene acetone compound represented by the formula II. The reaction for the formation of an enolate is carried out in an organic solvent such as tetrahydrofuran or diethyl ether at a temperature in the range of -70° to 0°C, preferably -30° to -10°C, for a period in the range of 5 minutes to 2 hours, preferably 10 to 30 minutes. Since the basic compounds mentioned above except for alkali metal alkoxides are not isolable, the formation of an enolate is generally effected by causing an alkyl amine to react with an alkyl alkali metal in the aforementioned organic solvent at a temperature in the range of -70° to 0°C, preferably -30° to -10°C, and having the aforementioned benzylidene acetone compound incorporated in the resultant reaction product. By allowing the resultant reaction solution to be reacted upon by an n-alkanoic acid imidazole amide at a temperature in the range of -100° to 0°C, preferably -80° to -50°C, there is obtained an unsaturated β-diketone. The n-alkanoic acid imidazole amides which are usable herein include butyric acid imidazole amide, caproic acid imidazole amide, capric acid imidazole amide, and caprylic acid imidazole amide, for example. The amount of the n-alkanoic acid imidazole amide to be used is in the range of 1 to 2 mols, preferably 1.1 to 1.5 mols, per mol of the unsaturated β-diketone. This reaction is carried out in an organic solvent mentioned above at a temperature in the range of -100° to 0°C, preferably -80° to -50°C, for a period in the range of 2 to 12 hours, preferably 3 to 4 hours. From the reaction product, the unsaturated β-diketone is obtained by extracting the reaction product, washing it with an acid, with an alkali, and then with a saturated common salt solution sequentially in the order mentioned, drying the washed reaction product, and distilling the dried product for expulsion of the solvent. The unsaturated β-diketone is treated in an organic solvent such as a methanol-tetrahydrofuran mixed solvent with a mineral acid such as hydrochloric acid, sulfuric acid, nitric acid, or phosphoric acid at a temperature in the range of 20° to 80°C, preferably 50° to 60°C, for a period in the range of 10 to 60 minutes, preferably 15 to 20 minutes to effect removal therefrom of a methoxymethyl group which is a protective group for a hydroxyl group and then is distilled for expulsion of the solvent and consequent crystallization of a catechol compound represented by the formula Ia.

A catechol compound represented by the formula Ib:

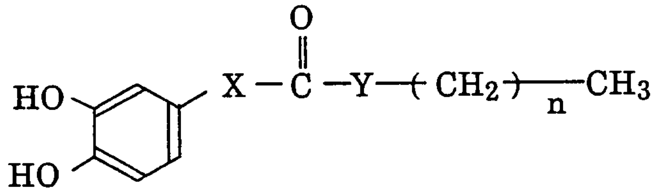

(Ib)

wherein X is $-CH_2-CH_2-$, Y is

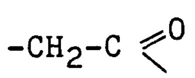 ,

and n is an integer in the range of 2 to 8, is obtained by causing a catechol compound represented by the formula Ia to react with hydrogen in the presence of a reducing catalyst. This hydrogenation is carried out in an organic solvent such as, for example, methanol, ethanol, or isopropanol at a temperature in the range of 0° to 50°c, preferably 20° to 30°C for a period in the range of 1 to 20 hours, preferably 5 to 15 hours. The reducing catalysts which are effectively usable herein include platinum, palladium, rhodium, palladium carbon, Raney nickel, and Raney cobalt, for example. This catalyst is used in an amount in the range of 0.1 to 100% by weight, preferably 5 to 20% by weight, based on the amount of a catechol compound of the formula Ia.

Then, a catechol compound represented by the formula Ic:

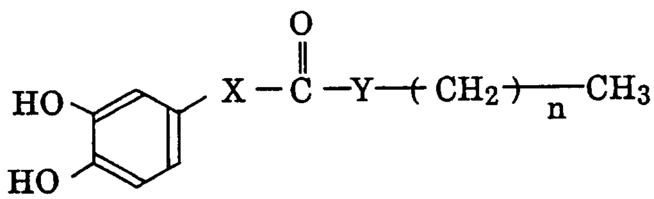

(Ic)

wherein X is -CH=CH-, Y is $-CH_2-CH(OH)-$, and n is an integer in the range of 2 to 8, is obtained by causing a benzylidene acetone compound represented by the formula II mentioned above to react with a basic compound by following the procedure employed for the production of a catechol compound represented by the formula Ia thereby forming an enolate, then causing the enolate to react with an n-alkane aldehyde having an alkyl group of 3 to 9 carbon atoms thereby forming an unsaturated $\beta$-hydroxy ketone, and treating the unsaturated $\beta$-hydroxy ketone with titanium tetrachloride.

The reaction with titanium tetrachloride is effected by dissolving titanium tetrachloride and the aforementioned enolate in an organic solvent such as chloroform and treating the resultant solution at a temperature in the range of -100° to 0°C, preferably -80° to -50°C, for a period in the range of 1 minute to 5 hours, preferably 15 minutes to 1 hour. The amount of titanium tetrachloride to be used herein is in the range of 50 to 200 parts by weight, preferably 60 to 150 parts by weight, based on 100 parts by weight of the aforementioned enolate.

Further, a catechol compound represented by the formula Id:

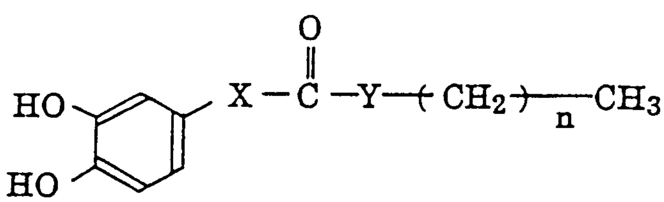

(Id)

wherein X is $-CH_2-CH_2-$, Y is $-CH_2-CH(OH)-$, and n is an integer in the range of 2 to 8, is obtained by causing a catechol compound represented by the aforementioned formula Ic to react with hydrogen in the presence of a reducing catalyst. The conditions for this reaction of hydrogenation are the same as those used in the production of a catechol compound represented by the formula Ib.

A catechol compound represented by the formula Ie:

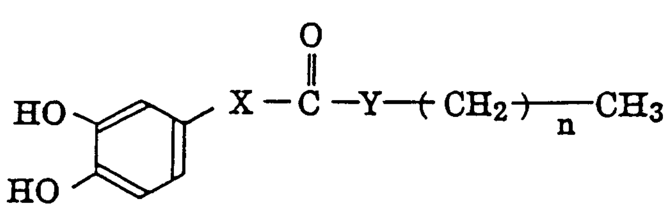

(Ie)

wherein X is $-CH_2-CH_2-$, Y is -CH=CH-, and n is an integer in the range of 2 to 8, is obtained by causing a catechol compound represented by the aforementioned formula Id to be dehydrated in the presence of an acid.

Further, a catechol compound represented by the formula If:

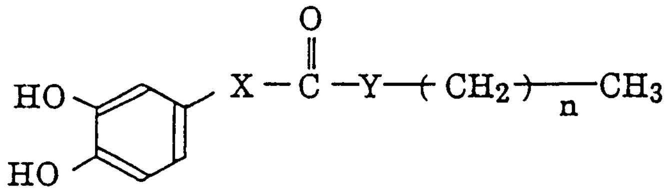

(If)

wherein X is $-CH_2-CH_2-$, Y is $-CH_2-CH_2-$, and n is an integer in the range of 2 to 8, is obtained by causing a catechol compound represented by the aforementioned formula Ie to react with hydrogen in the presence of a reducing catalyst.

The reaction of dehydration is carried out in an organic solvent such as methanol-benzene, ethanol benzene, methanol-toluene, or ethanol-toluene in the presence of an acid at a temperature in the range of 50° to 120°C, preferably at a refluxing temperature, for a period in the range of 1 to 5 hours, preferably 2 to 4 hours. The acids which are usable for this reaction include inorganic acids such as hydrochloric acid, sulfuric acid, nitric acid, and phosphoric acid and organic acids such as p-toluenesulfonic acid and acetic acid, for example. This acid is used in an amount in the range of 0.01 to 0.5 mol equivalent weight, preferably 0.05 to 0.1 mol equivalent weight, based on one mol of a catechol compound represented by the aforementioned formula Id. The reactions for hydrogenation of catechol compounds represented by the formula Ic and the formula Ie are carried out under the same conditions as those of the reaction for hydrogenation of a catechol compound represented by the formula Ia.

The catechol compounds of the present invention are used as a 5-lipoxygenase action inhibiting agent and an ulcer depressant. Though the dosage of these medicines is variable with the symptom of a disease under treatment, it is generally advisable to select a daily dosage for an adult in the range of 10 to 500 mg, preferably 20 to 200 mg, and administer the dosage, when necessary, as divided into two or three portions, depending on the symptom. This administration may be effected in any desired form. While the oral administration proves to be particularly desirable, the administration by intravenous injection is permissible.

The compound of the present invention, as a sole effective component or as one of a plurality of effective components, is prepared in numerous forms such as, for example, tablets, sugar-coated pills, powder, capsules, granules, suspension, emulsion, and injection either independently or as mixed with a pharmaceutical carrier or an excipient in accordance with the conventional method. The carriers and the excipients which are usable herein include calcium carbonate, calcium phosphate, starch, grape sugar, milk sugar, dextrin, alginic acid, mannitol, talc, and magnesium stearate, for example.

Now, the present invention will be described more specifically below with reference to working examples and test examples. It should be noted, however, that this invention is not limited in any respect to these examples.

Example 1

Under an atmosphere of argon gas, 6.11 g of diisopropyl amine was dissolved in 200 ml of dry tetrahydrofuran, the resultant solution was cooled to -15°C, and 40.2 ml of a hexane solution of 1.6 M n-butyl lithium was added dropwise thereto. The resultant mixture and a solution of 14.0 g of 4-(3,4-dimethoxymethyloxyphenyl)-3-buten-2-one in 50 ml of dry tetrahydrofuran added dropwise thereto at -15°C were stirred at the same temperature for 20 minutes. The mixture was cooled to -78°C. Then, the cooled mixture and a solution of 11.7 g of caprylic acid imidazole amide in 50 ml of dry tetrahydrofuran was added dropwise thereto were stirred at the same temperature for 3 hours and then at room temperature for 12 hours. The reaction solution was separated by the addition of a saturated aqueous solution of common salt into an organic layer and an aqueous layer. Then, the organic layer was extracted from the aqueous layer with ethyl acetate. The organic phase thus extracted was washed with a 2N hydrochloric acid, a saturated aqueous solution of sodium hydrogen carbonate, and a saturated aqueous solution of common salt sequentially in the order mentioned, dried with anhydrous magnesium sulfate, and then distilled under a vacuum to expel the solvent. The residue of the distillation was subjected to silica gel column chromatography. From the fraction eluted with methylene chloride, 14.6 g of 1-(3,4-dimethoxymethyloxyphenyl)-1-tetradecen-3,5-dione was obtained.

A solution of 7.44 g of the Claisen reaction adduct in 150 ml of methanol-tetrahydrofuran (1 : 1 V/V) and 15 ml of 6N hydrochloric acid added thereto were stirred at 60°C for 15 minutes. The resultant mixture was

distilled under a vacuum to expel the solvent and induce crystallization of the reaction product. When the crystals were separated by filtration and then washed with water, there was obtained 5.71 g of 1-(3,4-dihydroxyphenyl)-1-tetradecen-3,5-dione (III). The spectroscopic data of this compound support the structure of the following formula III.

$^1$HNMR(DMSO-$d_6$) δ: 0.83(3H,t,J = 4.5 Hz)
1.00-1.80(14H,m)
2.37(2H,t,J = 6.5 Hz)
5.78(1H,s)
6.43(1H,d,J = 15.5 Hz)
6.67-7.17(3H,m)
7.44(1H,d,J = 15.5 Hz)

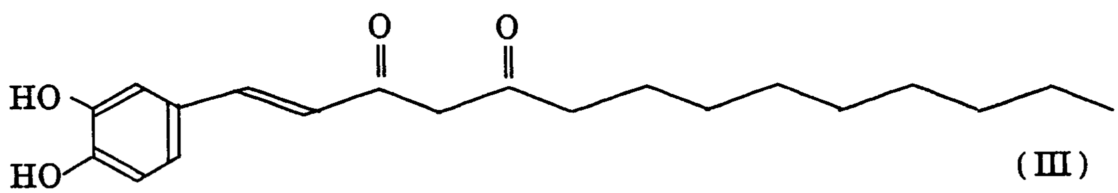

(III)

Example 2

A solution of 0.50 g of 1-(3,4-dihydroxyphenyl)-1-tetradecen-3,5-dione in 10 ml of methanol and 0.10 g of 5% palladium carbon added thereto were left reacting under an atmosphere of hydrogen gas for 5 hours. The resultant reaction mixture was filtered and then distilled under a vacuum to expel the solvent. The residue of the distillation was subjected to silica gel column chromatography. From the fraction obtained by elution with methylene chloride, 0.36 g of 1-(3,4-dihydroxyphenyl)-3,5-tetradecane dione (IV) was obtained. The spectroscopic data of this compound support the structure of the following formula IV.

$^1$HNMR(CDCℓ$_3$) δ: 0.87(3H,t,J = 5.0 Hz)
1.00-1.83(14H,m)
2.07-2.93(6H,m)
5.39(1H,s)
6.27-6.73(3H,m)

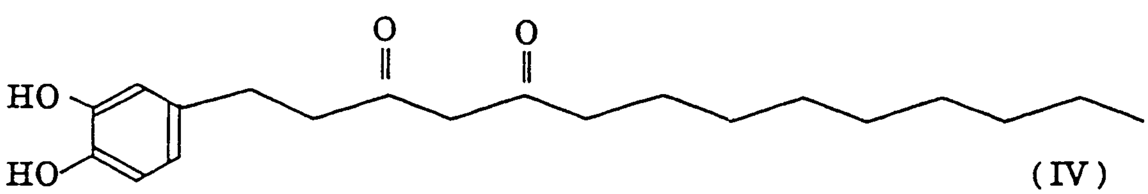

(IV)

Example 3

Under an atmosphere of argon gas, 8.74 g of diisopropyl amine was dissolved in 200 ml of dry tetrahydrofuran, the resultant solution was cooled to -15 °C, and 55.7 ml of a hexane solution of 1.6 M n-butyl lithium was added dropwise thereto. The resultant mixture and a solution of 19.2 g of 4-(3,4-dimethoxymethyloxyphenyl)-3-buten-2-one in 200 ml of dry tetrahydrofuran added dropwise thereto at -15 °C were stirred at the same temperature for 0.5 hour. The mixture was cooled to -78 °C. The cooled mixture and a solution of 13.6 g of capric aldehyde in 60 ml of dry tetrahydrofuran added dropwise thereto were stirred at the same temperature for 2.5 hours. The mixture and 30 ml of methanol added thereto were returned to room temperature and separated by addition of a saturated aqueous solution of common salt into an organic layer and an aqueous layer. The organic layer was extracted from the aqueous layer with diethyl ether. The organic phase thus separated was washed with 2N hydrochloric acid, a saturated aqueous solution of sodium hydrogen carbonate, and a saturated aqueous solution of common salt sequentially in the order mentioned, then dried with anhydrous magnesium sulfate, and distilled under a

vacuum to expel the solvent. The residue of the distillation was subjected to silica gel column chromatography. From the fraction eluted with methylene chloride-hexane (1 : 1 V/V), 27.8 g of 1-(3,4-dimethoxymethyloxyphenyl)-5-hydroxy-1-tetradecen-3-one was obtained.

A solution of 19.3 g of titanium tetrachloride in 50 ml of methylene chloride was cooled to -78°C. The cooled solution and a solution of 19.3 g of the aldol reaction adduct in 250 ml of methylene chloride added dropwise thereto were stirred at the same temperature for 0.5 hours. The resultant mixture was heated to room temperature and separated by the addition of 1500 ml of ethyl acetate and 500 ml of water into an organic layer and an aqueous layer. The organic layer was extracted from the aqueous layer with ethyl acetate. The organic phase thus separated was washed with a saturated aqueous solution of sodium hydrogen carbonate and a saturated aqueous solution of common salt sequentially in the order mentioned, dried with anhydrous magnesium sulfate, and distilled under a vacuum to expel the solvent. When the crude crystals obtained by the distillation were washed with ethanol-methylene chloride (3 : 97 V/V), there was obtained 8.33 g of 1-(3,4-dihydroxyphenyl-5-hydroxy-1-tetradecen-3-one (V). The spectrostopic data of this compound support the structure of the following formula V.

$^1$HNMR(DMSO-$d_6$) δ: 0.85(3H,t,J = 5 Hz)
1.03-1.67(16H,m)
2.63(2H,d,J = 6 Hz)
3.67-4.10(1H,m)
6.38(1H,d,J = 16 Hz)
6.67-7.10(3H,m)
7.33(1H,d,J = 16 Hz)

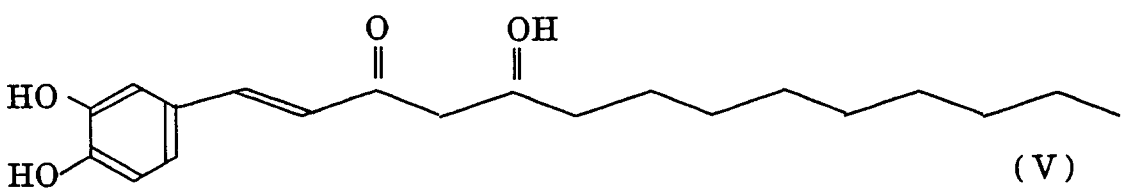

(V)

Example 4

A solution of 7.22 g of 1-(3,4-dihydroxyphenyl)-5-hydroxy-1-tetradecen-3-one in 200 ml of ethyl acetate and 1.00 g of 5% palladium carbon added thereto were left reacting under an atmosphere of hydrogen gas for 15 hours. The resultant reaction solution was filtered and then distilled under a vacuum to expel the solvent. The residue of the distillation was subjected to silica gel column chromatography. From the fraction eluted with methanol-methylene chloride (3 : 97 V/V), 5.94 g of 1-(3,4-hydroxyphenyl)-5-hydroxytetradecan-3-one (VI) was obtained. The spectroscopic data of this compound support the structure of the following formula VI.

$^1$HNMR($CDCl_3$) δ: 0.87(3H,t,J = 4.5 Hz)
1.07-1.62(16H,m)
2.47(2H,d,J = 6.0 Hz)
2.58-2.75(4H,m)
3.80-4.23(1H,m)
6.20-6.80(3H,m)

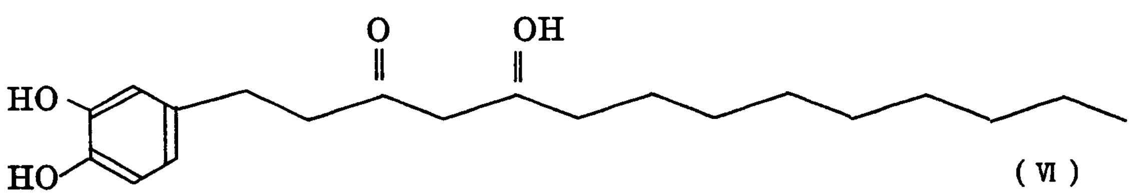

(VI)

Example 5

A solution of 3.30 g of 1-(3,4-dihydroxyphenyl)-5-hydroxytetradecan-3-oneand f0.30 g of p-toluenesulfonic acid monohydrate in 66 ml of methanol-benzene (1 : 10 V/V) was refluxed for 4 hours. The reaction solution consequently formed was separated by the addition of a saturated aqueous solution of sodium hydrogel carbonate into an organic layer and an aqueous layer. The organic layer was extracted from the aqueous layer with ethyl acetate. The organic phase thus separated was washed with a saturated aqueous solution of sodium hydrogen carbonate and a saturated aqueous solution of common salt sequentially in the order mentioned, dried with anhydrous magnesium sulfate, and then distilled under a vacuum to expel the solvent. The residue was subjected to silica gel column chromatography. From the fraction eluted with methanol-methylene chloride (1 : 9 V/V), 3.00 g of 1-(3,4-dihydroxyphenyl)-4-tetradecen-3-one (VII) was obtained. The spectroscopic data of this compound support the structure of the following formula VII.

$^1$HNMR($CDC\ell_3$) δ: 0.86(3H,t,J = 5 Hz)
1.03-2.38(16H,m)
2.60-2.90(4H,m)
6.08(1H,d,J = 16 Hz)
6.28-7.23(4H,m)

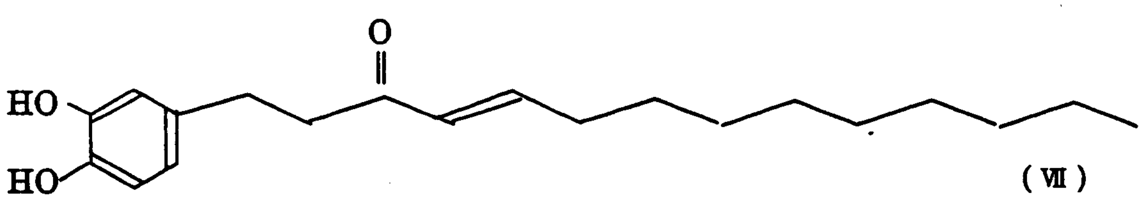

(VII)

Example 6

A solution of 0.37 g of 1-(3,4-dihydroxyphenyl)-4-tetradecen-3-one in 5 ml of methanol and 0.30 g of 5% palladium carbon added thereto were left reacting under an atmosphere of hydrogen gas for 25 hours. The resultant reaction mixture was filtered and distilled under a vacuum to expel the solvent. The residue of the distillation was subjected to silica gel column chromatography. From the fraction eluted with methanol-methylene chloride (5 : 95 V/V), 0.32 g of 1-(3,4-dihydroxyphenyl)-3-tetradecanone (VIII) was obtained. The spectroscopic data of this compound support the structure of the following formula VIII.

$^1$HNMR($CDC\ell_3$) δ: 0.87(3H,t,J = 6 Hz)
1.05-1.73(18H,m)
2.20-2.87(6H,m)
6.37-6.84(3H,m)

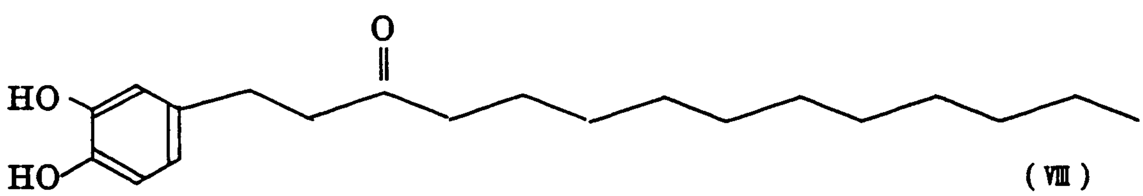

(VIII)

Example 7

Under an atmosphere of argon gas, 1.17 g of diisopropyl amine was dissolved in 30 ml of dry tetrahydrofuran, the resultant solution was cooled to -15 °C, and 7.50 ml of a hexane solution of 1.6 M n-butyl lithium was added dropwise thereto. The resultant mixture and a solution of 1.67 g of 4-(3,4-dimethoxymethyloxyphenyl)-3-buten-2-one in 10 ml of dry tetrahydrofuran added dropwise thereto at -15 °C were stirred at the same temperature for 20 minutes. The resultant mixture was cooled to -78 °C. The cooled mixture and a solution of 1.95 g of caprylic acid imidazole amide in 50 ml of dry tetrahydrofuran

added thereto dropwise were stirred at the same temperature for 3 hours and then at room temperature for 12 hours. The reaction solution thus obtained was separated by the addition of a saturated aqueous solution of common salt into an organic layer and an aqueous layer. The organic layer was extracted from the aqueous layer with ethyl acetate. The organic phase thus obtained was washed with 2N hydrochloric acid, a saturated aqueous solution of sodium hydrogen carbonate, and a saturated aqueous solution of common salt sequentially in the order mentioned, dried with anhydrous magnesium sulfate, and distilled under a vacuum to expel the solvent. The residue of the distillation was subjected to silica gel column chromatography. From the fraction eluated with methylene chloride-hexane (3 : 1 V/V), 2.07 g of 1-(3,4-dimethoxymethyloxyphenyl)-1-dodecen-3,5-dione was obtained.

A solution of 1.15 g of the Claisen reaction adduct in 20 ml of methanol-tetrahydrofuran (1 : 1 V/V) and 2.0 ml of 6N hydrochloric acid added thereto were stirred at 60oC for 20 minutes. The resultant mixture was distilled under a vacuum to expel the solvent and induce precipitation of crystals. When the crystals were separated by filtration and washed with water, there was obtained 0.81 g of 1-(3,4-dihydroxyphenyl)-1-dodecen-3,5-dione (IX). The spectroscopic data of this compound support the structure of the following formula IX.

$^1$HNMR(CDC$\ell_3$) $\delta$: 0.85(3H,t,J = 4.5 Hz)
1.0~1.8(10H,m)
2.34(2H,t,J = 6.5 Hz)
5.58H(1H,s)
6.23(1H,d,J = 15.5 Hz)
6.5~7.3(3H,m)
7.44(1H,d,J = 15.5 Hz)

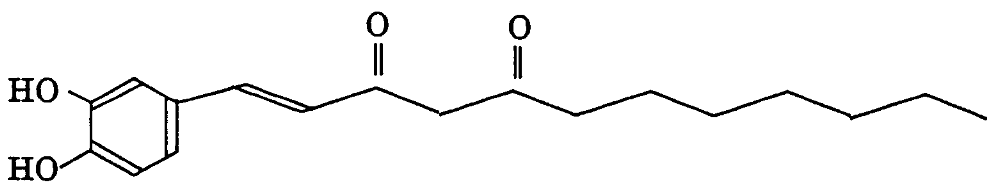

(IX)

Example 8

A solution of 300 mg of 1-(3,4-dihydroxyphenyl)-1-dodecen-3,5-dione in 6 ml of methanol and 0.10 g of 5% palladium carbon added thereto were left reacting under an atmosphere of hydrogen gas for 20 hours. The reaction mixture was filtered and then distilled under a vacuum to expel the solvent. The residue of the distillation was subjected to silica gel column chromatography. From the fraction eluted with methylene chloride, 157 mg of 1-(3,4-dihydroxyphenyl)-3,5-dodecane dione (X) was obtained. The spectroscopic data of this compound support the structure of the following formula X.

$^1$HNMR(CDC$\ell_3$) $\delta$: 0.86(3H,t,J = 4.5 Hz)
1.03~1.98(10H,m)
2.77~2.92(6H,m)
5.43(1H,s)
6.47~6.97(3H,m)

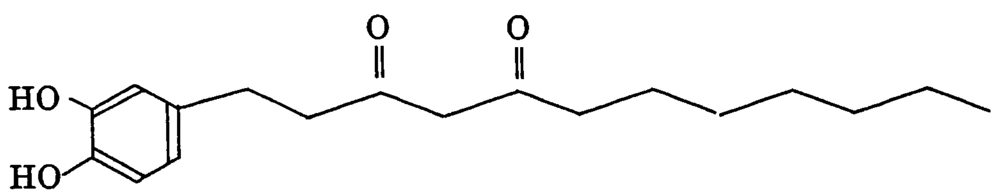

(X)

Example 9

By repeating the procedure of Example 7, excepting caproic acid imidazole amide was used instead, there was obtained 1-(3,4-dihydroxyphenyl)-1-decen-3,5-dione (XI) (38% in 2-step yield). The spectroscopic data of this compound support the structure of the following formula XI.

$^1$HNMR(CDC$\ell_3$-Methanol d$_4$;9:1) δ;
0.89(3H,t,J = 4.5 Hz)
1.05~1.83(6H,m)
2.39(2H,t,J = 6.5 Hz)
6.23(1H,d,J = 16 Hz)
6.50~7.15(3H,m)
7.42(1H,d,J = 16 Hz)

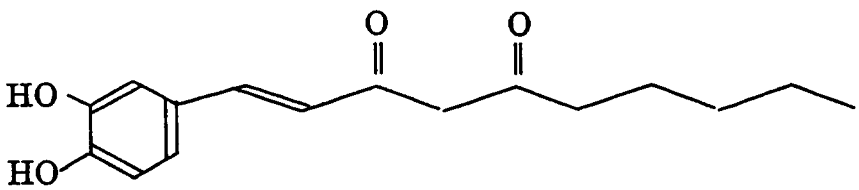

(XI)

Example 10

By repeating the procedure of Example 8, excepting 1-(3,4-dihydroxyphenyl)-1-decen-3,5-dione was used instead, there was obtained 1-(3,4-dihydroxyphenyl)-3,5-decane dione (XII) (54% in yield). The spectroscopic data of this compound support the structure of the following formula XII.

$^1$HNMR(CDC$\ell_3$) δ: 0.87(3H,t,J = 4.5 Hz)
1.0~1.93(6H,m)
2.0~3.0(6H,m)
5.38(1H,s)
6.23~6.79(3H,m)

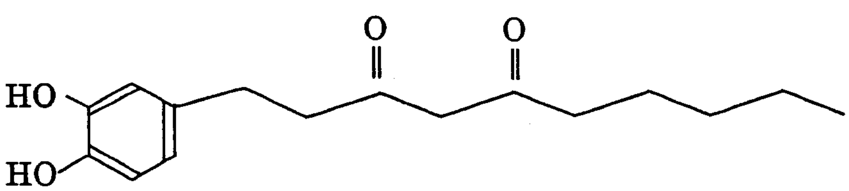

(XII)

Example 11

By repeating the procedure of Example 7, excepting n-butyric acid imidazole amide was used instead, there was obtained 1-(3,4-dihydroxyphenyl)-1-octen-3,5-dione (XIII) (43% in 2-step yield). The spectroscopic data of this compound support the structure of the following formula XIII.

$^1$HNMR(DMSOd$_6$) δ: 0.91(3H,t,J = 7 Hz)
1.59(2H,sex,J = 7 Hz)
2.34(2H,t,J = 7 Hz)
5.81(1H,s)
6.40(1H,d,J = 15.5 Hz)
6.60-7.14(3H,m)
7.39(1H,d,J = 15.5 Hz)

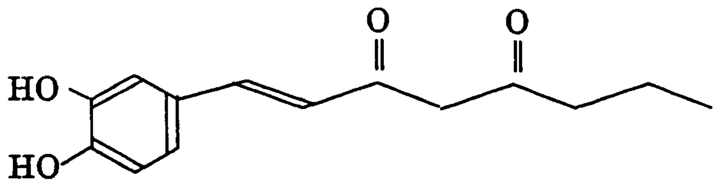

(XIII)

Example 12

By repeating the procedure of Example 8, excepting 1-(3,4-dihydroxyphenyl)-1-octen-3,5-dione was used instead, there was obtained 1-(3,4-dihydroxyphenyl)-3,5-octadione (XIV) (62% in yield). The spectroscopic data of this compound support the structure of the following formula XIV.

$^1$HNMR(CDCℓ$_3$) δ: 0.92(3H,t,J = 6 Hz)
1.14-3.0(8H,m)
5.46(1H,s)
6.34-6.90(3H,m)

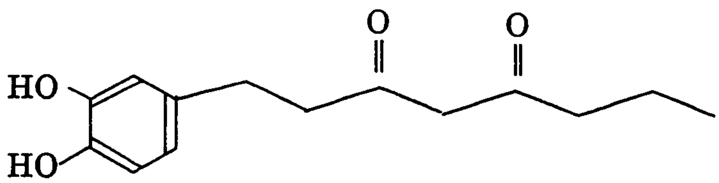

(XIV)

[Test Example]

(1) Activity to inhibit 5-lipoxygenase action

A rat-originating basophilic leukemic cell strain, RBL-1, was suspended in a culture solution having 10% FSC contained in a basic culture medium (produced by Gibco Laboratories and marketed under trademark designation of "Eagle") and cultured in a 5% $CO_2$ incubator at 37°C. The culture broth consequently formed was centrifuged at 4°C to collect cells. The were re-suspended in a phosphate buffer solution of pH 7.4 to a cell density in the range of 1.0 × $10^7$ to 3.0 x $10^7$ cells/ml. The suspended cells were treated with an ultrasonic cell crushing device and then centrifuged at 15,000 rpm at 4°C for 30 minutes. The superntant was taken as a 5-lipoxygenase solution. In a test tube, 50 μg of arachidonic acid and a catechol compound of this invention given to be tested were placed and the resultant mixture and 0.30 ml of a phosphate buffer solution, 0.20 ml of the enzyme solution mentioned above, and 5 ul of a 100 mM $CaCl_2$ -(calcium chloride) solution added thereto were left reacting at 37°C for 15 minutes. The reaction mixture consequently formed was cooled with ice and, with one drop of 1N HCl (hydrochloric acid) added thereto, extracted from 2 ml of ethyl acetate. The extract was concentrated to dryness and then combined with 100 μl of methanol, to produce a sample.

This sample was injected into an octadecyl silane (ODS) type reverse-phase high-speed liquid chromatographic (HPLC) system. The adsorbate was eluted with a 15 : 45 : 35 : 0.01 methanol : acetonitrile : water : acetic acid solvent to determine the peak height of a 5-HETE (5-(s)-hydroxy-6,8,11,14-eicosatetraeic acid), a 5-lipoxygenase product. The activity of the sample to inhibit the 5-lipoxygenase action was confirmed by a decrease in the peak height of the 5-lipoxygenase product mentioned above. As the result of the test, the compounds of the present invention were found to exhibit conspicuous activities in inhibiting the 5-lipoxygenase action as shown in Table 1 below. The catechol compounds according with the present invention which are not shown in Table 1 were conformed to exhibit similar activities in inhibiting the 5-lipoxygenase action.

Table 1

| Activities to inhibit 5-lipoxygenase action | | |
|---|---|---|
| Example | Structural formula | Concentration (mol) for 50% inhibition |
| 1 | III | $3.3 \times 10^{-7}$ |
| 2 | IV | $4.5 \times 10^{-7}$ |
| 3 | V | $4.2 \times 10^{-7}$ |
| 4 | VI | $3.3 \times 10^{-7}$ |
| 5 | VII | $3.2 \times 10^{-7}$ |
| 6 | VIII | $1.6 \times 10^{-7}$ |
| 7 | IX | $1.2 \times 10^{-7}$ |
| 8 | X | $2.0 \times 10^{-7}$ |
| 9 | XI | $6.2 \times 10^{-7}$ |
| 10 | XII | $1.7 \times 10^{-7}$ |
| 11 | XII | $1.9 \times 10^{-7}$ |
| 12 | XIV | $3.8 \times 10^{-7}$ |

The term "concentration for 50% inhibition" as used in the table refers to the concentration of a given catechol compound required for inhibiting the formation of the 5-HETE, i.e. the 5-lipoxygenase product, from 100% registered in the absence of the catechol compound to 50% registered in the presence of the added catechol compound.

(2) Activity to resist growth of ulcer

A Wistar type male rat (body weight 150 to 200 g) was fasted for 24 hours, then orally administered with a catechol compound according with the present invention and, on elapse of 1 hour thereafter, orally administered with ethanol hydrochloric acid (having 150 mM hydrochloric acid contained in 60% ethanol) at a rate of 0.5 ml/100 g of body weight.

After 1 hour following the oral administration of ethanol hydrochloric acid, the rat was sacrified with ether. The stomach was excited from the corpse, treated with formalin, and visually examined to measure lengths of injuries generated on the glanular part of the stomach. The total of injuries was reported as an ulcer index of the rat in question.

The results are shown in Table 2. It is clearly noted from this table that the catechol compounds according with the present invention exhibited conspicuous activities to resist growth of ulcer. The catechol compounds according with the present invention which are not shown in Table 2 were confirmed to exhibit similar activities in resisting the growth of ulcer.

Table 2

| Activity on ulcer | | |
|---|---|---|
| Example No. | Amount administered | Ratio of inhibiting formation of ulcer (%) |
| 1 | 30mg/kg | 82.4 |
| 2 | 30mg/kg | 92.1 |
| 4 | 30mg/kg | 84.4 |
| 7 | 30mg/kg | 85.7 |
| 8 | 30mg/kg | 88.3 |
| 9 | 30mg/kg | 77.9 |
| 10 | 30mg/kg | 84.6 |
| 11 | 30mg/kg | 58.7 |
| 12 | 30mg/kg | 81.8 |

The term "ratio of inhibiting formation of ulcer (%)" as used in the table refers to a numerical value obtained by subtracting the ulcer index of a rat orally administered with a given catechol compound

according with the present invention from the ulcer index of a rat not orally administered with catechol compound and multiplying the difference by 100.

[Acute toxicity]

ICR type male mice (5 weeks old) were used for testing catechol compounds according with the present invention for acute toxicity due to oral administration. The LD50 values consequently found for the compounds of this invention were invariably not more than 2,000 mg/kg, indicating high safety relative to the amount of efficacy.

Industrial Applicability

The present invention provides a novel catechol compound, a method for the production thereof, and a 5-lipoxygenase action inhibiting agent and an ulcer depressant both containing the compound.

The compound of this invention mentioned above has been demonstrated to possess an activity to inhibit a 5-lipoxygenase action and an activity to resist growth of ulcer. To be specific, this compound, owing to the activity to inhibit the action of 5-lipoxygenase, is capable of curbing the occurrence of leucotrienes such as $LTC_4$ and $LTD_4$ which are formed by the action of 5-lipoxygenase. The catechol compound, therefore, can be used effectively as a 5-lipoxygenase action inhibiting agent for curing gastritis, hepatitis, rheumatism, gastric ulcer, etc. as well as such allergic diseases as asthma and rhinitis.

Further, since the compound of the present invention is capable of curbing ulcers, it can be used effectively as a medicine for the cure of gastric ulcer, for example.

## Claims

**1.** A catechol compound represented by the formula I:

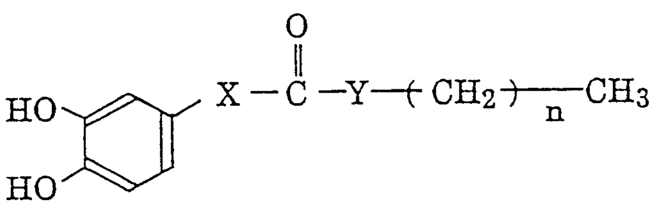

(I)

wherein X is one group selected from the class consisting of $-CH_2-CH_2-$ and $-CH=CH-$, Y is one group selected from the class consisting of $-CH=CH-$ and $-CH_2-CZ-$ (where Z is $=O$ or 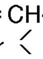), and n is an integer in the range of 2 to 8.

**2.** A catechol compound according to claim 1, wherein Y is

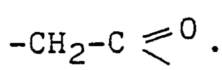

**3.** A catechol compound according to claim 1, wherein Y is the group $-CH_2-CH(OH)-$ and n is 8.

**4.** A method for the production of a catechol compound represented by the formula Ia:

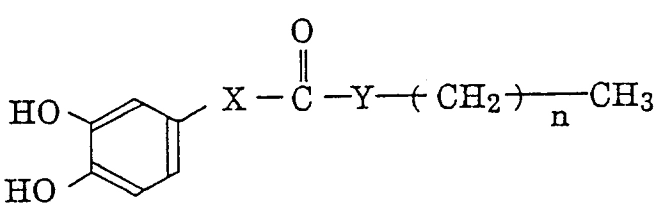

(Ia)

wherein X is -CH = CH-, Y is

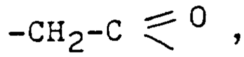

and n is an integer in the range of 2 to 8, which method comprises causing a benzylidene acetone compound represented by the formula II:

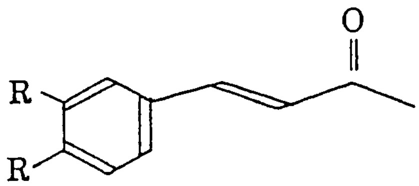

(II)

wherein R is methoxymethyloxy group, to react with at least one basic compound selected from the group consisting of alkali metal dialkyl amides, alkali metal dicycloalkyl amides, alkali metal alkoxides, alkyl alkali metals, and alkali metal hexamethyl silazide thereby forming an enolate, subjecting said enolate to a reaction with an n-alkanoic acid imidazole amide having an alkyl group of 3 to 9 carbon atoms thereby forming an unsaturated $\beta$-diketone, and treating said unsaturated $\beta$-diketone with a mineral acid.

**5.** A method for the production of a catechol compound represented by the formula Ib:

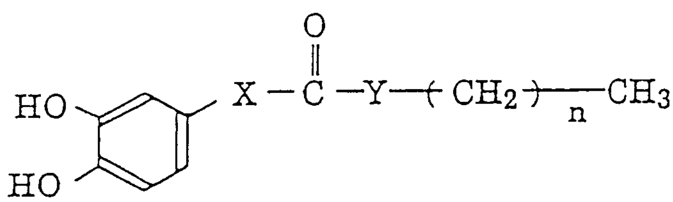

(Ib)

wherein X is $-CH_2-CH_2-$, Y is

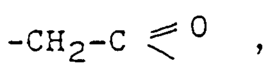

and n is an integer in the range of 2 to 8, which method comprises causing a catechol compound represented by the formula Ia set forth in claim 4 to react with hydrogen in the presence of a reducing catalyst.

**6.** A method for the production of a catechol compound represented by the formula Ic:

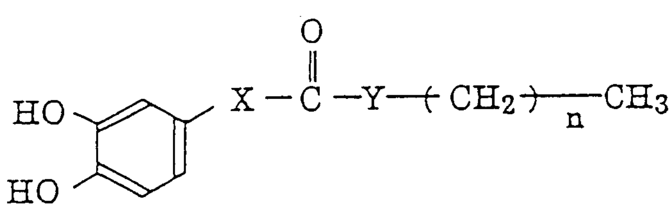

(Ic)

wherein X is -CH = CH-, Y is $-CH_2-CH(OH)-$, and n is an integer in the range of 2 to 8, which method comprises causing a benzylidene acetone compound represented by the formula II:

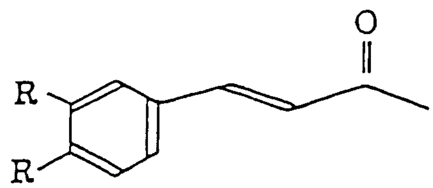

(II)

wherein R is methoxymethyloxy group, to react with at least one basic compound selected from the group consisting of alkali metal dialkyl amides, alkali metal dicycloalkyl amides, alkali metal alkoxides, alkyl alkali metals, and alkali metal hexamethyl silazide thereby forming an enolate, subjecting said enolate to a reaction with an n-alkane aldehyde thereby forming an unsaturated $\beta$-hydroxy ketone, and treating said unsaturated $\beta$-hydroxy ketone with titanium tetrachloride.

**7.** A method for the production of a catechol compound represented by the formula Id:

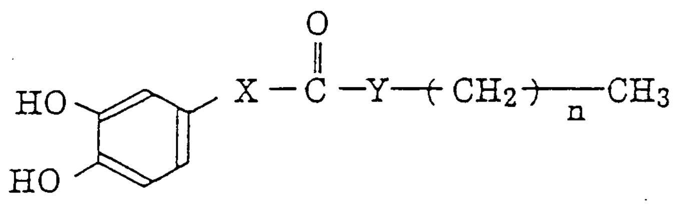

(Id)

wherein X is $-CH_2-CH_2-$, Y is $-CH_2-CH(OH)-$, and n is an integer in the range of 2 to 8, which method comprises causing a catechol compound represented by the formula Ic set forth in claim 6 to react with hydrogen in the presence of a reducing catalyst.

**8.** A method for the production of a catechol compound represented by the formula Ie:

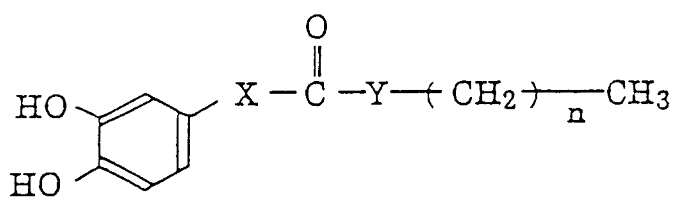

(Ie)

wherein X is $-CH_2-CH_2-$, Y is $-CH=CH-$, and n is an integer in the range of 2 to 8, which method comprises dehydrating a catechol compound represented by the formula Id set forth in claim 7 in the presence of an acid.

**9.** A 5-lipoxygenase action inhibiting agent, having as an effective component thereof a catechol compound set forth in claim 1.

**10.** A 5-lipoxygenase action inhibiting agent according to claim 9, wherein Y is

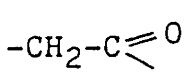 .

**11.** A 5-lipoxygenase action inhibiting agent according to claim 9, wherein Y is one group selected from the class consisting of $-CH_2-CH(OH)-$, $-CH=CH-$, and n is 8.

**12.** An ulcer depressant, having as an active component thereof a catechol compound set forth in claim 1.

**13.** An ulcer depressant according to claim 12, wherein Y is

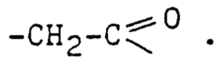

**14.** An ulcer depressant according to claim 12, wherein Y is one group selected from the class consisting of $-CH_2-CH(OH)-$ and $-CH=CH-$ and n is 8.

**15.** The use of the catechol derivative represented by the formula I

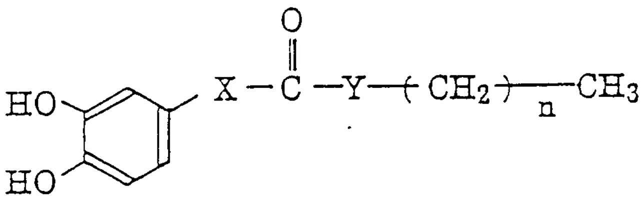

in which :
X is $-CH_2-CH_2-$ or $-CH=CH-$, Y is $-CH_2-CH_2-$ and n is an integer in the range of 2 to 8 for the preparation of a medicament useful for depressing ulcers.

**16.** The use according to claim 15, of compounds of formula (I) in which Y is $-CH_2-CH_2-$ and n is 8.

**Patentansprüche**

**1.** Brenzkatechinverbindung der Formel I

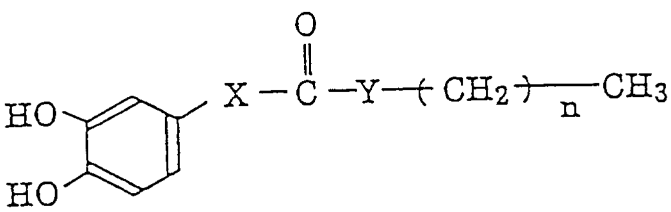

(I)

worin bedeuten:

X $-CH_2-CH_2-$ oder $-CH=CH-$
Y $-CH=CH-$ oder $-CH_2-CZ-$ mit Z gleich $=O$ oder
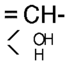 und
n eine ganze Zahl im Bereich von 2 bis 8.

**2.** Brenzkatechinverbindung nach Anspruch 1, worin Y für

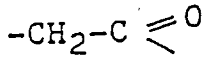

steht.

**3.** Brenzkatechinverbindung nach Anspruch 1, worin Y für die Gruppe $-CH_2-CH(OH)-$ steht und n = 8 ist.

**4.** Verfahren zur Herstellung einer Brenzkatechinverbindung der Formel Ia:

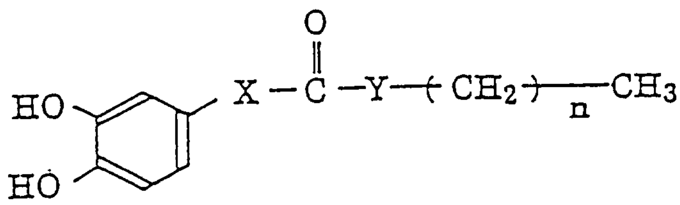

(Ia)

worin bedeuten:

X -CH = CH-;

Y

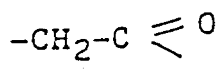

und

n eine ganze Zahl im Bereich von 2 bis 8,

durch Reagierenlassen einer Benzylidenacetonverbindung der Formel II:

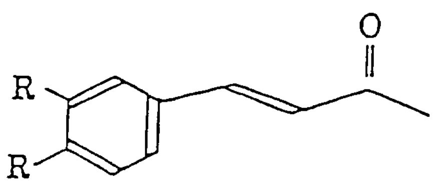

(II)

worin R für eine Methoxymethyloxygruppe steht, mit mindestens einer basischen Verbindung, ausgewählt aus der Gruppe Alkalimetalldialkylamide, Alkalimetalldicycloalkylamide, Alkalimetallalkoxide, Alkylalkalimetalle und Alkalimetallhexamethylsilazid zur Bildung eines Enolats, Umsetzen des Enolats mit einem N-Alkancarbonsäureimidazolamid mit einer Alkylgruppe mit 3 bis 9-Kohlenstoffatomen zur Bildung eines ungesättigten $\beta$-Diketons und Behandeln des ungesättigten $\beta$-Diketons mit einer Mineralsäure.

**5.** Verfahren zur Herstellung einer Brenzkatechinverbindung der Formel Ib:

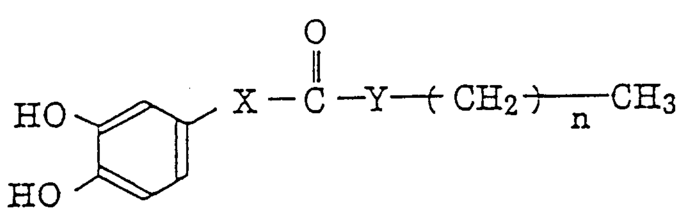

(Ib)

worin bedeuten:

X $-CH_2-CH_2-$;

Y

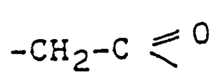

und

n eine ganze Zahl im Bereich von 2 bis 8,
durch Reagierenlassen einer Brenzkatechinverbindung der Formel Ia nach Anspruch 4 mit Wasserstoff in Gegenwart eines Reduktionskatalysators.

**6.** Verfahren zur Herstellung einer Brenzkatechinverbindung der Formel Ic:

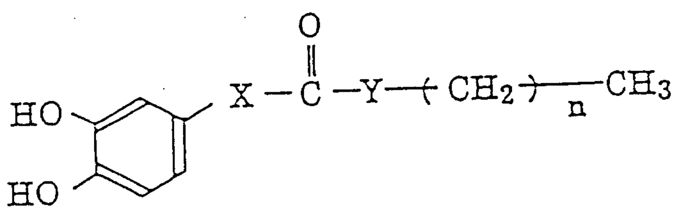

(Ic)

worin bedeuten:
X -CH=CH-;
Y $-CH_2-CH(OH)-$ und
n eine ganze Zahl im Bereich von 2 bis 8,
durch Reagierenlassen einer Benzylidenacetonverbindung der Formel II:

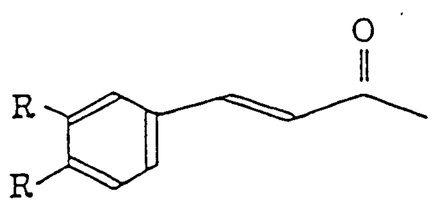

(II)

worin R für eine Methoxymethyloxygruppe steht, mit mindestens einer basischen Verbindung, ausgewählt aus der Gruppe Alkalimetalldialkylamide, Alkalimetalldicycloalkylamide, Alkalimetallalkoxide, Alkylalkalimetalle und Alkalimetallhexamethylsilazid, zur Bildung eines Enolats, Umsetzen des Enolats mit einem N-Alkanaldehyd unter Bildung eines ungesättigten $\beta$-Hydroxyketons und Behandeln des ungesättigten $\beta$-Hydroxyketons mit Titantetrachlorid.

**7.** Verfahren zur Herstellung einer Brenzkatechinverbindung der Formel Id:

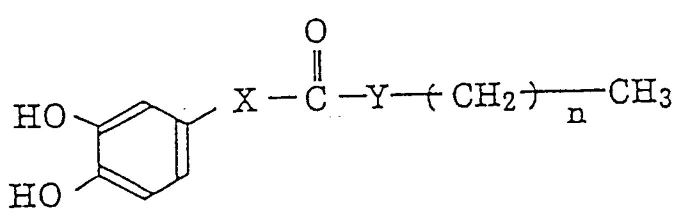

(Id)

worin bedeuten:
X $-CH_2-CH_2-$;
Y $-CH_2-CH(OH)-$ und
n eine ganze Zahl im Bereich von 2 bis 8,
durch Reagierenlassen einer Brenzkatechinverbindung der Formel Ic von Anspruch 6 mit Wasserstoff in Gegenwart eines Reduktionskatalysators.

**8.** Verfahren zur Herstellung einer Brenzkatechinverbindung der Formel Ie:

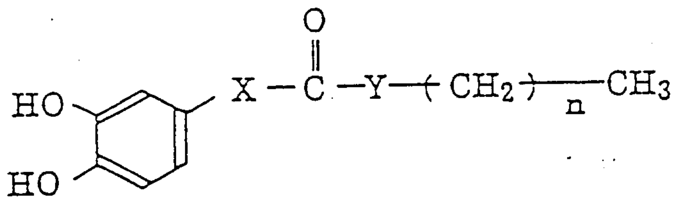

(Ie)

worin bedeuten:

X $-CH_2-CH_2-$;

Y $-CH=CH-$ und

n eine ganze Zahl im Bereich von 2 bis 8,

durch Dehydratisieren einer Brenzkatechinverbindung der Formel Id von Anspruch 7 in Gegenwart eines Säures.

**9.** Die Wirkung von 5-Lipoxygenase hemmendes Mittel mit einer Brenzkatechinverbindung nach Anspruch 1 als wirksamer Komponente.

**10.** Die Wirkung von 5-Lipoxygenase hemmendes Mittel nach Anspruch 9, worin Y für

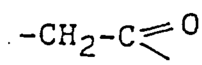

steht.

**11.** Die Wirkung von 5-Lipoxygenase hemmendes Mittel nach Anspruch 9, worin Y für $-CH_2-CH(OH)-$ oder $-CH=CH-$ steht und n = 8 ist.

**12.** Ein Ulkusbildung hemmendes Mittel mit einer Brenzkatechinverbindung nach Anspruch 1 als aktiver Komponente.

**13.** Ein Ulkusbildung hemmendes Mittel nach Anspruch 12, worin Y für

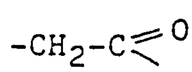

steht.

**14.** Ein Ulkusbildung hemmendes Mittel nach Anspruch 12, worin Y für $-CH_2-CH(OH)-$ oder $-CH=CH-$ steht und n = 8 ist.

**15.** Verwendung des Brenzkatechinderivats der Formel I

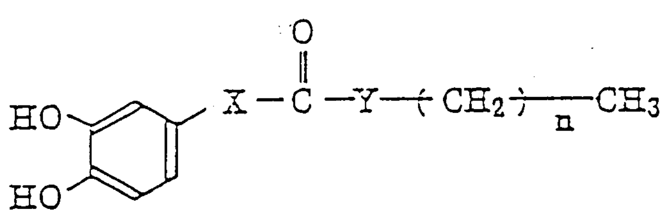

worin bedeuten:

X -$CH_2$-$CH_2$- oder -CH = CH-;

Y -$CH_2$-$CH_2$- und

n eine ganze Zahl im Bereich von 2 bis 8,

zur Herstellung eines Medikaments zur Ulkusunterdrückung.

**16.** Verwendung nach Anspruch 15 von Verbindungen der Formel (I), worin Y für -$CH_2$-$CH_2$- steht und n = 8 ist.

## Revendications

**1.** Composé de catéchol représenté par la formule I:

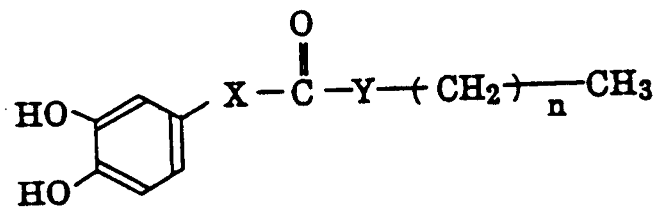

(I)

dans laquelle X représente un groupe choisi parmi -$CH_2$-$CH_2$- et -CH = CH-, Y représente un groupe choisi parmi -CH = CH- et -$CH_2$-CZ- (où Z est = O ou $<^{OH}_{H}$ ), et n est un nombre entier compris entre 2 et 8.

**2.** Composé de catéchol selon la revendication 1, dans lequel Y représente

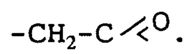

**3.** Composé de catéchol selon la revendication 1, dans lequel Y représente le groupe -$CH_2$-CH(OH)- et n vaut 8.

**4.** Procédé pour la production d'un composé de catéchol représenté par la formule Ia:

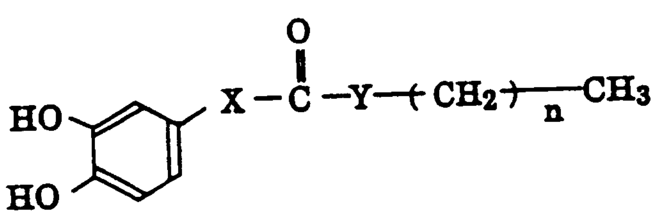

(Ia)

dans laquelle X représente -CH = CH-, Y représente

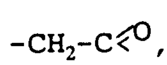

et n est un nombre entier de 2 à 8, procédé qui consiste à faire réagir un composé de benzylidène acétone représenté par la formule II:

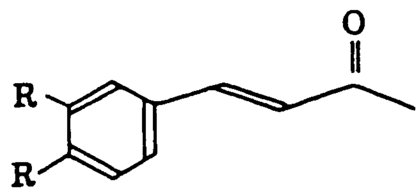

(II)

dans laquelle R représente un groupe méthoxyméthyloxy, avec au moins un composé basique choisi dans le groupe formé par les dialkylamides de métal alcalin, les dicycloalkylamides de métal alcalin, les alcoxydes de métal alcalin, les alkylures de métal alcalin, et l'hexaméthyl-silazide de métal alcalin formant ainsi un énolate, à soumettre ledit énolate à une réaction avec un imidazole amide d'acide n-alcanoïque ayant un groupe alkyle de 3 à 9 atomes de carbone, formant ainsi une $\beta$-dicétone insaturée, et à traiter ladite $\beta$-dicétone insaturée avec un acide minéral.

**5.** Procédé pour la production d'un composé de catéchol représenté par la formule Ib:

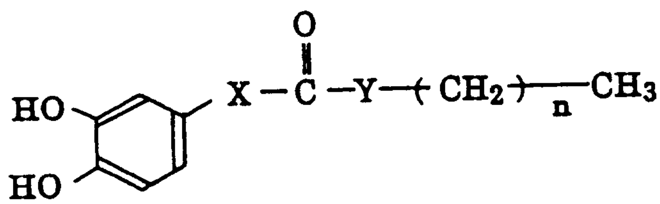

(Ib)

dans laquelle X représente $-CH_2-CH_2-$, Y représente

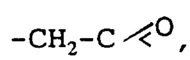

et n est un nombre entier de 2 à 8, procédé qui consiste à faire réagir un composé de catéchol représenté par la formule Ia décrit dans la revendication 4 avec de l'hydrogène en présence d'un catalyseur de réduction.

**6.** Procédé pour la production d'un composé de catéchol représenté par la formule Ic:

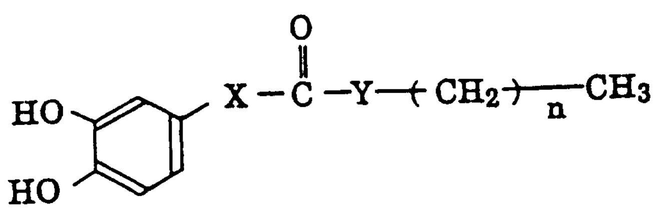

(Ic)

dans laquelle X représente $-CH=CH$, Y représente $-CH_2-CH(OH)-$, et n est un nombre entier de 2 à 8, procédé qui consiste à faire réagir un composé de benzylidène acétone représenté par la formule II:

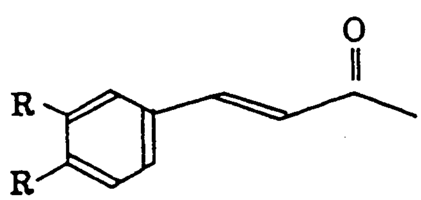

(II)

dans laquelle R représente un groupe méthoxyméthyloxy, avec au moins un composé basique choisi dans le groupe formé par les dialkylamides de métal alcalin, les dicycloalkylamides de métal alcalin,

les alcoxydes de métal alcalin, les alkylures de métal alcalin, et l'hexaméthyl-silazide de métal alcalin formant ainsi un énolate, à soumettre ledit énolate à une réaction avec un aldéhyde de n-alcane formant ainsi une β-hydroxy-cétone insaturée, et à traiter ladite β-hydroxy-cétone insaturée avec du tétrachlorure de titane.

**7.** Procédé pour la production d'un composé de catéchol représenté par la formule Id:

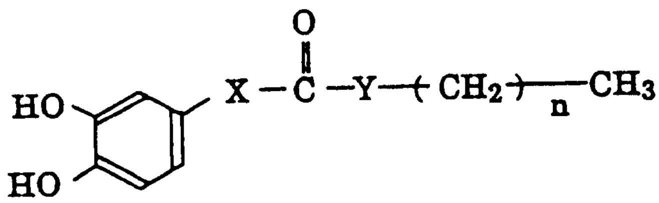

(Id)

dans laquelle X représente $-CH_2-CH_2-$, Y représente $-CH_2-CH(OH)-$, et n est un nombre entier de 2 à 8, procédé qui consiste à faire réagir un composé de catéchol représenté par la formule Ic décrit dans la revendication 6 avec de l'hydrogène en présence d'un catalyseur de réduction.

**8.** Procédé pour la production d'un composé de catéchol représenté par la formule Ie:

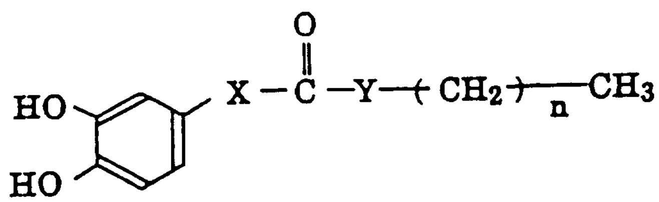

(Ie)

dans laquelle X représente $-CH_2-CH_2-$, Y représente -CH = CH-, et n est un nombre entier de 2 à 8, procédé qui consiste à déshydrater un composé de catéchol représenté par la formule Id décrit dans la revendication 7 en présence d'un acide.

**9.** Agent inhibant l'action de la 5-lipoxygénase, ayant comme composant efficace, un composé de catéchol décrit dans la revendication 1.

**10.** Agent inhibant l'action de la 5-lipoxygénase selon la revendication 9, dans lequel Y représente

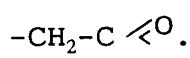

**11.** Agent inhibant l'action de la 5-lipoxygénase selon la revendication 9, dans lequel Y représente un groupe choisi parmi $-CH_2-CH(OH)-$ et -CH = CH-, et n vaut 8.

**12.** Modérateur d'ulcère, ayant comme composant actif, un composé de catéchol décrit dans la revendication 1.

**13.** Modérateur d'ulcère selon la revendication 12, dans laquelle Y représente

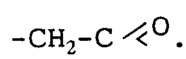

**14.** Modérateur d'ulcère selon la revendication 12, dans lequel Y représente un groupe choisi parmi $-CH_2-CH(OH)-$ et -CH = CH- et n vaut 8.

**15.** Utilisation du dérivé de catéchol représenté par la formule I

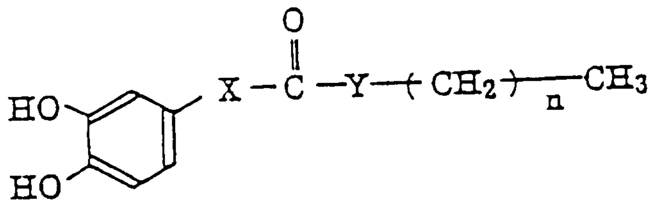

dans laquelle: X représente $-CH_2-CH_2-$ ou $-CH=CH-$, Y représente $-CH_2-CH_2-$ et n est un nombre entier de 2 à 8, pour la préparation d'un médicament utile pour modérer les ulcères.

**16.** Utilisation selon la revendication 15 de composés de formule (I) dans laquelle Y représente $-CH_2-CH_2-$ et n vaut 8.